# FASCICULE DE BREVET EUROPEEN

(11) **EP 0 832 637 B1**
(45) Date de publication et mention de la délivrance du brevet: **27.10.1999**
(21) Numéro de dépôt: 97401933.3
(22) Date de dépôt: 13.08.1997
(51) Int. Cl.: A61K 7/032

(54) **Compositions pour le maquillage des yeux**
Kosmetische Zusammensetzung von Augenschminke
Cosmetic eye make up composition

(30) Priorité: 30.09.1996 FR 9611878
(43) Date de publication de la demande: 01.04.1998
(73) Titulaire: L'OREAL, 75008 Paris (FR)
(72) Inventeur: Collin, Nathalie, 92330 Sceaux (FR); Couture, Huguette, 91270 Vigneux sur Seine (FR)
(74) Mandataire: Lhoste, Catherine

(56) Documents cités:
- EP-A- 0 610 026
- EP-A- 0 687 461
- WO-A-91/12793
- WO-A-94/17775
- WO-A-95/15741

## Description

La présente invention concerne une composition, notamment pour le maquillage des yeux, comprenant des dérivés siliconés oxyalkylénés. Ces compositions présentent des propriétés cosmétiques, notamment de tenue, améliorées.

Il existe sur le marché de nombreuses formulations de mascara. Toutefois, le consommateur attend toujours une amélioration des propriétés de ces mascaras que sont l'allongement du ci, une meilleure adhésion de la composition sur le ci, une meilleure mise en forme, appelée aussi empesage, des cils, une meilleure tenue du maquillage dans le temps et une meilleure résistance aux sollicitations mécaniques. Par exemple, certaines utilisatrices souhaitent pouvoir conserver le même maquillage pour la journée et la soirée. On sait aussi que le frottement des doigts sur les paupières et le contact des lunettes sur les cils ont tendance à faire s'effriter le mascara. En particulier, la présence de pigments et/ou de charges dans les compositions pour le maquillage des cils rend ces compositions particulièrement sujettes à l'effritement. Les compositions connues jusqu'alors ne résolvaient pas ce problème de façon satisfaisante.

Le document WO-A-91/12793 décrit un mascara résistant à l'eau comprenant une cire, un agent de consistance, un solvant organique volatil et une solution aqueuse de polymère filmogène hydrosoluble. Le document EP-A-0687461 décrit un mascara comprenant une cire et une dispersion aqueuse de polymère constituée de particules issues de la polymérisation d'un monomère radicalaire en présence de particules de polymère choisi parmi les polyesters, les polyesteramides et les alkydes.

La présente invention a pour but de proposer une composition, notamment pour le maquillage des yeux, présentant une rapidité de séchage, des propriétés d'allongement et d'empesage des cils, de tenue et de résistance mécanique améliorées.

L'invention a pour objet une composition cosmétique, notamment pour le maquillage des cils, caractérisée en ce qu'elle comprend :
(a) au moins une phase aqueuse comprenant un polymère filmogène,
(b) au moins une phase grasse comprenant au moins une cire, et
(c) un agent siliconé choisi parmi :
   (i) les silicones oxyalkylénées, et/ou
   (ii) les copolymères blocs linéaires polysiloxane-polyoxyalkylène.

Ces compositions se présentent préférentiellement sous la forme d'une émulsion de la phase grasse dans la phase aqueuse ou d'une dispersion de la phase aqueuse dans la phase grasse.

On connaissait jusqu'à présent les diméthicones copolyols dans différentes applications : comme agent émulsionnant dans des compositions du type eau-dans-huile, ou encore comme agent plastifiant dans des produits de coiffage des cheveux. Par exemple on pourra se référer au document EP-A-705595 qui décrit des compositions pour le coiffage des cheveux comprenant un agent plastifiant, qui peut être un diméthicone copolyol ou un acide polycarboxylique, pour améliorer la flexibilité des résines fixantes.

Toutefois, les compositions décrites dans ce document sont très différentes par leur structure des compositions pour le maquillage des yeux. En particulier, ces compositions ne comportent pas de pigments ni de charges, elles ne comprennent pas non plus de quantités élevées de cires. A la lecture de ce document, rien n'incitait l'homme du métier à penser que les propriétés d'allongement du ci, d'adhésion sur le cil, d'empesage des cils, de tenue du maquillage et de résistance aux sollicitations mécaniques des mascaras, pouvaient être améliorées de façon spectaculaire par l'utilisation de diméthicones copolyols.

Avantageusement, les compositions selon l'invention comportent en outre au moins un pigment, qui peut être choisi parmi des pigments minéraux, organiques éventuellement nacrés. Ces pigments sont de préférence compris en une proportion allant de 0,25 à 25%, de préférence de 1 à 20%, en poids par rapport au poids total de la composition, suivant la coloration et l'intensité de la coloration que l'on cherche à obtenir. Parmi les pigments susceptibles d'être utilisés, on peut citer les oxydes de zinc, de fer ou de chrome, le bleu ferrique, le noir de carbone, et les laques couramment employées qui sont des sels de colorants acides.

La composition selon l'invention comprend donc un agent siliconé qui peut être une silicone oxyalkylénée.

Les silicones oxyalkylénées sont choisies parmi les composés de formule générale (1) appelés aussi diméthicone copolyols : 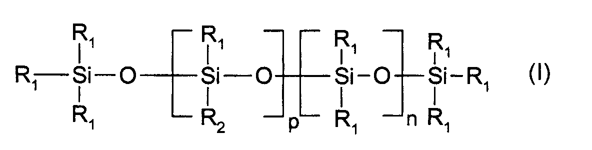 formule dans laquelle :
- R₁, identiques ou différents, représentent un atome d'hydrogène, un radical alkyle linéaire ou ramifié en C₁-C₃₀ ou un radical phényle,
- R₂, identiques ou différents, représentent -(CₓH₂ₓ)-(OC₂H₄)ₐ-(OC₃H₆)_{b}-OR₃,
- R₃, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 0 à 1000,
- p varie de 1 à 30,
- a varie de 0 à 50,
- b varie de 0 à 50,
- a + b est supérieur ou égal à 1,
- x varie de 1 à 5,
- le poids moléculaire moyen en nombre étant supérieur ou égal à 15000 et de préférence compris entre 25000 et 75000.

De façon préférentielle, on utilise les silicones oxyalkylénées de formule générale (I) qui répondent à au moins une des, et de préférence à toutes les, conditions suivantes :
- R₁ désigne le radical méthyle.
- R₃ représente un atome d'hydrogène, un radical méthyle ou un radical acétyle, et de préférence hydrogène.
- p varie de 8 à 20.
- a est compris entre 5 et 40 et de préférence entre 15 et 30.
- b est compris entre 5 et 40 et de préférence entre 15 et 30.
- x est égal à 2 ou 3.
- n varie de 20 à 600, de préférence de 50 à 500 et encore plus particulièrement de 100 à 300.

De telles silicones sont par exemple décrites dans le brevet US-4,311,695 qui est inclus à titre de référence.
Des diméthicones copolyols ont en particulier été présentés par la société DOW CORNING lors du 17ème congrès international de l'I.F.S.C.C. d'octobre 1992 et rapportés dans l'article "Water-soluble dimethicone copolyol waxes for personal care industry" de Linda Madore et al., pages 1 à 3.
Ces diméthicones copolyols sont des polydiméthylsiloxanes (PDMS) comportant une ou plusieurs fonctions éthers, solubles dans l'eau (oxyalkylène, notamment oxyéthylène et/ou oxypropylène).

De tels diméthicones copolyols sont notamment vendus par la société GOLDSCHMIDT sous la dénomination ABIL B8851 ou ABIL B88183. On peut citer aussi les composés KF 351 à 354 et KF 615 A vendus par la société SHIN ETSU ou la DMC 6038 de la société WACKER.
Les dérivés de diméthicones copolyols utilisables dans l'invention sont en particulier les diméthicones copolyols à groupement phosphate, sulfate, chlorure de myristamide propyldiméthylammonium, stéarate, amine, glycomodifié, etc. On peut utiliser comme dérivés de diméthicones copolyols notamment les composés vendus par la société SILTECH sous la dénomination Silphos A100, Siltech amine 65, Silwax WDIS, myristamido silicone quat, ou par la société PHOENIX sous la dénomination Pecosil PS 100.
On peut également utiliser les dérivés vendus par la société WACKER sous la dénomination VP 1661, ou par la société DOW CORNING sous la dénomination 2501 cosmetic wax. On peut encore utiliser les dérivés vendus par la société WACKER sous la dénomination VP 1661, ou par la société DOW CORNING sous la dénomination 2501 cosmetic wax.
Les silicones les plus particulièrement préférées sont par exemple celles vendues par la société DOW CORNING sous la dénomination commerciale Q2-5220 et par la société RHONE POULENC sous la dénomination MIRASIL DMCO.

En particulier, les silicones oxyalkylénées selon l'invention ne contiennent pas de groupement alkyle ou alcoxy ayant de 8 à 22 atomes de carbone et lié directement à un atome de silicium.

Lorsque l'on utilise, dans le cadre de la présente invention, des copolymères blocs linéaires polysiloxane-polyoxyalkylène, ceux-ci ont de préférence la formule générale (II) suivante : 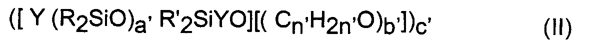 dans laquelle :
- R et R' identiques ou différents, représentent un radical hydrocarboné monovalent ne contenant pas d'insaturation aliphatique,
- n 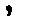 est un nombre entier compris entre 2 et 4,
- a 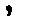 est un nombre entier supérieur ou égal à 5,
- b 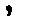 est un nombre entier supérieur ou égal à 4,
- c 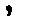 est un nombre entier supérieur ou égal à 4,
- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10% environ à 90% environ en poids du copolymère bloc,
- le poids moléculaire moyen du copolymère bloc étant d'au moins 3.000.

Les radicaux R et R' sont plus préférentiellement choisis dans le groupe comprenant les radicaux alkyle comme par exemple les radicaux méthyle, éthyle, propyle, butyle, pentyle, hexyle, octyle, décyle, dodécyle; les radicaux aryle comme par exemple phényle, naphtyle; les radicaux aralkyle comme par exemple benzyle, phényléthyle; les radicaux tolyle, xylyle et cyclohexyle.
Le radical divalent Y est de préférence -R''-, -R''-CO-, -R''-NHCO-, -R''-NH-CO-NH-R'''-NHCO ou -R''-OCONH-R'''-NHCO-, où R'' est un groupe alkylène divalent comme par exemple l'éthylène, le propylène ou le butylène et R''' est un groupe alkylène divalent ou un groupe arylène divalent comme -C₆H₄-, -C₆H₄-C₆H₄-, -C₆H₄-CH₂-C₆H₄- ou -C₆H₄-CH(CH₃)₂-C₆H₄-.
Encore plus préférentiellement Y représente un radical alkylène divalent, plus particulièrement le radical -CH₂-CH₂-CH₂-.
La préparation des copolymères blocs mis en oeuvre dans le cadre de la présente invention est notamment décrite dans la demande européenne EP 0 492 657 A1.

Selon un mode de réalisation particulier de l'invention le copolymère bloc est choisi parmi les copolymères suivants :
[[(CH₃)₂SiO]₄₁(CH₃)₂SiCH₂CH(CH₃)CH₂-O(C₂H₄O)₁₈- (C₃H₆O)₃₃CH₂CH(CH₃)CH₂]_{16.1}
[[(CH₃)₂SiO]₃₁(CH₃)₂SiCH₂CH(CH₃)CH₂-O(C₂H₄O)₂₀- (C₃H₆O)₂₉CH₂CH(CH₃)CH₂]_{13.3}
[[(CH₃)₂SiO]₉(CH₃)₂SiCH₂CH(CH₃)CH₂-O(C₂H₄O)₂₀- (C₃H₆O)₂₉CH₂CH(CH₃)CH₂]_{26.3}
[[(CH₃)₂SiO]₁₆(CH₃)₂SiCH₂CH(CH₃)CH₂-O(C₂H₄O)₁₈- (C₃H₆O)₂₀CH₂CH(CH₃)CH₂]_{21.5}
[[(CH₃)₂SiO]₉(CH₃)₂SiCH₂CH(CH₃)CH₂-O(C₂H₄O)₅-CH₂CH(CH₃)CH₂]_{4.8}

Les agents siliconés utilisés dans les compositions de l'invention peuvent être hydrosolubles ou liposolubles. Suivant leur hydro- ou lipo-solubilité, ils sont introduits respectivement dans la phase aqueuse ou la phase grasse.

Les cires susceptibles d'être utilisées dans la composition selon l'invention possèdent en règle générale un point de fusion compris entre 40 et 110 °C et ont une pénétration à l'aiguille, à 25 °C, comprise entre 3 et 40, telle que mesurée selon la norme américaine ASTM D 5 ou selon la norme française NFT 004. Le principe de la mesure de pénétration d'une aiguille selon les normes ASTM D 5 et NFT 004 consiste à mesurer la profondeur exprimée en dixième de millimètres, à laquelle pénètre une aiguille normalisée qui pèse 2,5 g placée dans un porte-aiguilles pesant 47,5 g soit un total de 50 g, l'aiguille étant placée sur la cire pendant 5 secondes.
Les cires utilisables dans la présente invention peuvent être choisies parmi les cires animales, les cires végétales, les cires minérales, les cires synthétiques et les fractions diverses de cires naturelles, toutes les cires présentant les deux caractéristiques physiques mentionnées précédemment.
Parmi les cires animales, on peut citer les cires d'abeille, les cires de lanoline et les cires d'insectes de Chine.
Parmi les cires végétales, on peut citer les cires de riz, les cires de Carnauba, de Candellila, d'Ouricurry, les cires de fibres de liège, les cires de canne à sucre, les cires du Japon, la cire de Sumac, la cire de coton.
Parmi les cires minérales, on peut citer les paraffines, les cires microcristallines, les cires de Montan et les ozokérites.
Parmi les cires synthétiques, on peut utiliser notamment les cires de polyéthylène, les cires obtenues par la synthèse de Fischer et Tropsch, les copolymères cireux ainsi que leurs esters, les cires de silicone.
Il est également possible d'utiliser des huiles animales ou végétales hydrogénées qui répondent toujours aux deux caractéristiques physiques mentionnées précédemment.
Parmi ces huiles, on peut citer les cires de jojoba hydrogénées et les huiles hydrogénées qui sont obtenues par hydrogénation catalytique de corps gras composés de chaîne grasse linéaire ou non en C₈-C₃₂, l'huile de tournesol hydrogénée, l'huile de ricin hydrogénée, l'huile de coprah hydrogénée, la lanoline hydrogénée et les huiles de palme hydrogénées.
Les cires utilisables selon la présente invention sont de préférence solides et rigides à température inférieure à 50 °C.
Ces cires peuvent se présenter sous forme de dispersions stables de particules colloïdales de cire telles qu'elles peuvent être préparées selon des méthodes connues, telles que "Microémulsions Theory and Practice", L. M. Prince Ed., Academic Pross (1977), pages 21-32.

Avantageusement, les compositions selon l'invention comprennent de 6 à 40% de cire, en poids par rapport au poids total de la composition, et préférentiellement de 10 à 25%.

La composition selon l'invention comprend en outre au moins un polymère filmogène, en solution ou en dispersion dans la phase aqueuse.

Le polymère filmogène est utilisé en quantités qui permettent d'obtenir une bonne adhésion de la composition sur le cil, un allongement, un gainage et une courbure qui donnent une impression d'ouverture du regard, les cils étant bien séparés.
De façon préférentielle, les compositions selon l'invention comprennent une quantité de polymère filmogène allant de 0,1 à 25% en poids par rapport au poids total de la composition. Avantageusement, les compositions qui font l'objet de la présente invention comprennent une quantité allant de 1 à 10%, en poids par rapport au poids total de la composition, de polymère filmogène.

Le polymère filmogène peut être choisi parmi :
- les dérivés de kératine, tels que les hydrolysats de kératine et les kératines sulfoniques ;
- les dérivés de chitine ou de chitosane anioniques, cationiques, amphotères ou non-ioniques ;
- les dérivés de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polyvinylpyrrolidones et les copolymères vinyliques, tels que les copolymères de l'éther méthylvinylique et de l'anhydride malique, ou le copolymère de l'acétate de vinyle et de l'acide crotonique ;
- les polymères polyester et/ou polyesteramide anioniques dispersables dans l'eau, comprenant des monomères portant une fonction: -SO₃M, avec M représentant un atome d'hydrogène, un ion ammonium NH₄⁺ ou un ion métallique, comme par exemple un ion Na⁺, Li⁺, K⁺, Mg²⁺, Ca²⁺, Cu²⁺, Fe²⁺, Fe³⁺. On peut citer en particulier les polymères décrits dans les documents US-3,734,874 ; US-4,233,196 ; US-4,304,901.

Avantageusement, on choisit des polymères polyesters filmogènes à base d'au moins un acide dicarboxylique, d'au moins un diol et d'au moins un monomère aromatique bifonctionnel portant en outre un groupement -SO₃M tel que décrit ci-dessus.
- les polyesters à chaîne grasse, les polyamides, et les résines époxyesters.
- les polymères polyuréthannes, notamment les polyuréthannes anioniques, cationiques, non ioniques ou amphotères, les polyuréthannes-acryliques, les polyuréthannes-polyvinylpirrolidones, les polyester-polyuréthannes, les polyéther-polyuréthannes, les polyurées, les polyurée/polyuréthannes, et leurs mélanges.
- les polymères d'origine naturelle, éventuellement modifiés, tels que :
   . les gommes arabiques, la gomme de guar, les dérivés du xanthane, la gomme de karaya ;
   . les alginates et les carraghénates ;
   . les glycoaminoglycanes, l'acide hyaluronique et ses dérivés ;
   . la résine shellac, la gomme de sandaraque, les dammars, les élémis, les copals.

De préférence les compositions selon l'invention comprennent de 0,01 à 5% d'agent siliconé et encore plus préférentiellement de 0,4 à 1,5%, en poids par rapport au poids total de la composition. Avantageusement, les compositions selon l'invention comprennent de 10 à 30% d'agent siliconé, en poids par rapport au poids total de polymère filmogène.

Les composants des phases huileuse et aqueuse peuvent être indépendamment dissous ou fondus à une température de 85 °C puis mélangés.

La composition selon la présente invention peut se présenter sous forme d'une émulsion huile-dans-eau ou d'une dispersion eau-dans-cire.

Lorsqu'elle est utilisée sous forme d'émulsion huile-dans-eau, la composition peut contenir des agents tensioactifs émulsionnants présents en une proportion comprise entre 2 et 30 % en poids par rapport au poids total de la composition. Ces agents tensioactifs peuvent être choisis parmi des agents tensioactifs anioniques ou non ioniques. On peut se reporter au document 〈〈 Encyclopedia of Chemical Technology, KIRK-OTHMER 〉〉, volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans les compositions selon l'invention sont :
- parmi les tensioactifs non-ioniques: les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés.
- parmi les tensioactifs anioniques : les stéarates d'amines.

Lorsque la composition selon l'invention est sous la forme d'une émulsion huile-dans-eau, l'eau représente avantageusement de 30 à 80% en poids du poids total de la composition.

Lorsque la composition selon l'invention est sous la forme d'une dispersion eau-dans-cire, elle comprend en outre de préférence au moins un agent épaississant et au moins un solvant ou une huile organique volatil qui s'évaporeront au contact de la peau ou des cils, mais dont la présence dans la composition cosmétique est utile car ils facilitent l'étalement de la composition lors de l'application sur la peau ou les cils. De tels agents d'étalement appelés ici solvants ou huiles volatiles sont généralement des composés organiques ayant à 25°C une tension de vapeur saturante au moins égale à 0,5 millibar (soit 0,5.10²Pa).

L'agent épaississant peut être choisi parmi les argiles modifiées organiquement, tels que les montmorillonites et les dérivés d'hectorite, par exemple la bentonite.

La concentration en solvant ou en huile organique volatil est comprise entre 35 et 70% en poids par rapport au poids total de la composition. Le solvant ou l'huile organique volatile peut être choisi parmi l'isoparaffine, l'essence de térébenthine, l'alcool isopropylique, l'alcool éthylique, le white spirit, les huiles de silicone telles que l'hexaméthyldisiloxane, le cyclopentadiméthylsiloxane, le cyclotétraméthylsiloxane, les huiles fluorées comme celles commercialisées sous la dénomination Galden (Montefluos) ou les huiles isoparaffiniques telles que celles qui sont commercialisées sous la dénomination ISOPAR (E, G, L ou H) ainsi que l'isododecane.

Dans cette forme de réalisation de l'invention, la phase aqueuse représente de préférence de 0,1 à 25% en poids par rapport au poids total de la composition.

On peut se reporter au document WO91/12793 pour la préparation de compositions sous la forme d'une dispersion eau-dans-cire, ces dispersions ayant en outre la propriété d'être résistantes à l'eau.

La composition selon l'invention peut également contenir des ingrédients couramment utilisés en cosmétique, tels que les vitamines, les oligo-éléments, les adoucissants, les séquestrants, les parfums, les huiles, les silicones, les agents de cohésion ainsi que les agents alcalinisants ou acidifiants habituellement utilisés dans le domaine cosmétique, les charges, les émollients, habituellement utilisés en quantités comprises entre 1 et 10%; les conservateurs comme par exemple l'imidazolinyl urée, le méthylparaben, le propylparaben ; des composés fluorés.

La composition peut en outre comprendre au moins une charge, telle que :
- le talc qui est un silicate de magnésium hydraté utilisé sous forme de particules généralement inférieures à 40 microns,
- les micas qui sont des aluminosilicates de compositions variées se présentant sous la forme d'écailles ayant des dimensions de 2 à 200 microns, de préférence de 5 à 70 microns et une épaisseur comprise entre 0,1 à 5 microns, de préférence de 0,2 à 3 microns, ces micas pouvant être d'origine naturelle telle que la muscovite la margarite, la roscoelithe, la lipidolithe, la biotite ou d'origine synthétique,
- l'amidon en particulier l'amidon de riz,
- le kaolin qui est un silicate d'aluminium hydraté qui se présente sous la forme de particules de forme isotrope ayant des dimensions généralement inférieures à 30 microns,
- les oxydes de zinc et de titane généralement utilisés sous la forme de particules ayant des dimensions ne dépassant pas quelques microns,
- le carbonate de calcium, le carbonate ou l'hydrocarbonate de magnésium,
- la cellulose microcristalline,
- la silice,
- les poudres de polymères synthétiques tels que le polyéthylène, les polyesters (l'isophtalate ou le téréphtalate de polyéthylène), les polyamides tels que ceux vendus sous la dénomination commerciale de "Nylon" ou de "Téflon" et les poudres de silicone.

Les compositions selon l'invention peuvent notamment se présenter sous la forme d'un mascara ou d'un eye-liner, ou constituer une base pour la préparation de mascaras ou d'eye-liners.

L'invention a également pour objet l'utilisation d'une composition telle que définie ci-dessus, pour l'obtention d'un maquillage présentant une meilleure tenue dans le temps et/ou un empesage amélioré et/ou une meilleure résistance à l'effritement et/ou un meilleur allongement des cils et/ou une vitesse de séchage améliorée.

L'invention a également pour objet l'utilisation d'un agent siliconé choisi parmi :
(i) les silicones oxyalkylénées, et/ou
(ii) les copolymères blocs linéaires polysiloxane-polyoxyalkylène,
   dans une composition cosmétique, notamment de maquillage des yeux, comprenant :
   (a) au moins une phase aqueuse comprenant un polymère filmogène, et
   (b) au moins une phase grasse comprenant au moins une cire,
      pour conférer à cette composition une meilleure tenue dans le temps et/ou un empesage amélioré et'ou une meilleure résistance à l'effritement et/ou un meilleur allongement des cils et/ou une vitesse de séchage améliorée.

### EXEMPLES

Dans les exemples tous les pourcentages sont donnés en poids de matière active.

Les mascaras sont évalués par des utilisatrices (6 personnes) pour leur rapidité de séchage, leur adhérence immédiate sur le ci, leur tenue dans le temps, leur résistance au frottement (l'utilisatrice se frotte les paupières avec les doigts et constate l'état du maquillage après frottement), l'allongement et l'empesage qu'ils confèrent aux cils.

### Exemple 1 : une composition de mascara selon l'invention est préparée en faisant un mélange homogène à partir des ingrédients suivants :

| | |
|---|---|
| Cire d'abeille | 3 % |
| Cire de Carnauba | 3 % |
| Paraffine | 13 % |
| Oxyde de fer noir | 7 % |
| Stéarate de triéthanolamine | 9 % |
| Gomme arabique | 3,5 % |
| Hydroxyéthylcellulose | 1 % |
| Polydiméthylsiloxane oxyéthyléné (*) | 0,6 % |
| Polyméthacrylate de sodium | 0,25 % |
| Conservateurs | qs |
| Eau | qsp |

| | |
|---|---|
| (*) commercialisé par la société Dow Corning sous la référence commerciale Q2-5220 Cette composition est jugée par les utilisatrices comme satisfaisant à tous les critères de choix : il adhère bien sur le cil, sèche rapidement, il allonge le cil et l'empèse, il a une très bonne tenue et une excellente résistance aux frottements (aucun effritement constaté). | |

### Exemple 2 (comparatif) :

| | |
|---|---|
| Cire d'abeille | 3 % |
| Cire de Carnauba | 3 % |
| Paraffine | 13 % |
| Oxyde de fer noir | 7 % |
| Stéarate de triéthanolamine | 9 % |
| Gomme arabique | 3,5 % |
| Hydroxyéthylcellulose | 1 % |
| Polyméthacrylate de sodium | 0,25 % |
| Conservateurs | qs |
| Eau | qsp |

Par mélange homogène de tous les composants on obtient une composition de mascara. Deux heures après application, ce mascara est évalué par les utilisatrices comme satisfaisant mais inférieur au mascara de l'exemple 1 en rapidité de séchage, allongement du cil et surtout en empesage des cils, en tenue dans le temps et en résistance aux frottements (un peu d'effritement constaté).

### Exemple 3 (comparatif) :

| | |
|---|---|
| Cire d'abeille | 3 % |
| Cire de Carnauba | 3 % |
| Paraffine | 13 % |
| Oxyde de fer noir | 7 % |
| Stéarate de triéthanolamine | 9 % |
| Gomme arabique | 3,5 % |
| Hydroxyéthylcellulose | 1 % |
| Acétyl citrate de tri-éthyle | 0,6 % |
| Polyméthacrylate de sodium | 0,25 % |
| Conservateurs | qs |
| Eau | qsp |

Par mélange homogène de tous les composants on obtient une composition de mascara. Deux heures après application, ce mascara est évalué par les utilisatrices: il est jugé comme ayant une mauvaise tenue. La résistance au frottement est très insuffisante. Le mascara s'effrite en formant des copeaux.

L'acétyl citrate de tri-éthyle est connu comme agent plastifiant des résines, de même que certaines silicones oxyalkylénées, toutefois, il n'a pas les propriétés des silicones oxyalkylénées dans les compositions selon l'invention.

### Exemple 4 (comparatif) : une composition de mascara selon l'invention est préparée en faisant un mélange homogène à partir des ingrédients suivants :

| | |
|---|---|
| Cire d'abeille | 3 % |
| Cire de Carnauba | 3 % |
| Paraffine | 13 % |
| Oxyde de fer noir | 7 % |
| Stéarate de triéthanolamine | 9 % |
| Polydiméthylsiloxane oxyéthyléné (*) | 4,75 % |
| Conservateurs | qs |
| Eau | qsp |

| | |
|---|---|
| (*) commercialisé par la société Dow Corning sous la référence commerciale Q2-5220 On constate par cet exemple qu'en l'absence de polymère filmogène (remplacé par son équivalent poids en Q2-5220) on obtient une composition ayant une viscosité équivalente à celle de l'eau, qui ne peut pas être utilisée pour du maquillage. En outre, les pigments sont très mal dispersés. | |

## Revendications

1. Composition cosmétique, caractérisée en ce qu'elle comprend :
(a) au moins une phase aqueuse comprenant un polymère filmogène,
(b) au moins une phase grasse comprenant au moins une cire,
(c) un agent siliconé choisi parmi :
(i) les silicones oxyalkylénées,
(ii) les copolymères blocs linéaires polysiloxane-polyoxyalkylène.

2. Composition selon la revendication précédente, caractérisée en ce qu'elle est sous **la** forme d'une émulsion de la phase grasse dans la phase aqueuse ou d'une dispersion de la phase aqueuse dans la phase grasse.

3. Composition selon l'une quelconque des revendications précédentes, caractérisée en ce qu'elle comprend en outre de 0,25 à 25 %, de préférence de 1 à 20%, en poids par rapport au poids total de la composition d'au moins un pigment.

4. Composition selon l'une quelconque des revendications précédentes, caractérisée en ce que la silicone oxyalkylénée est choisie parmi les composés de formule générale (I): 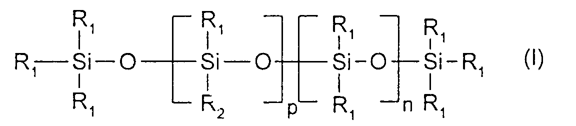 dans laquelle :
- R₁, identiques ou différents, représentent un atome d'hydrogène, un radical alkyle linéaire ou ramifié en C₁-C₃₀ ou un radical phényle,
- R₂, identiques ou différents, représentent -(CₓH₂ₓ)-(OC₂H₄)ₐ-(OC₃H₆)_{b}-OR₃,
- R₃, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 0 à 1000,
- p varie de 1 à 30,
- a varie de 0 à 50,
- b varie de 0 à 50,
- a + b est supérieur ou égal à 1,
- x varie de 1 à 5,
le poids moléculaire moyen en nombre étant supérieur ou égal à 15.000 et de préférence compris entre 25.000 et 75.000.

5. Composition selon la revendication précédente, caractérisée en ce qu'elle comprend une silicone oxyalkylénée de formule générale (I) qui répondent à au moins une des, et de préférence à toutes les, conditions suivantes :
- R₁ désigne le radical méthyle.
- R₃ représente un atome d'hydrogène, un radical méthyle ou un radical acétyle et de préférence hydrogène.
- p varie de 8 à 20.
- a est compris entre 5 et 40 et de préférence entre 15 et 30.
- b est compris entre 5 et 40 et de préférence entre 15 et 30.
- x est égal à 2 ou 3.
- n varie de 20 à 600, de préférence de 50 à 500 et encore plus particulièrement de 100 à 300.

6. Composition selon l'une quelconque des revendications précédentes, caractérisé en ce que le copolymère bloc linéaire polysiloxane-polyoxyalkylène répond à la formule (II) suivante : 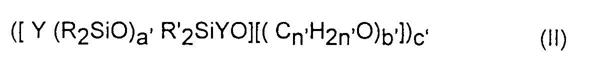 dans laquelle :
- R et R' identiques ou différents, représentent un radical hydrocarboné monovalent ne contenant pas d'insaturation aliphatique,
- n 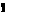 est un nombre entier compris entre 2 et 4,
- a 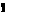 est un nombre entier supérieur ou égal à 5,
- b 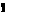 est un nombre entier supérieur ou égal a 4,
- c 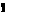 est un nombre entier supérieur ou égal à 4,
- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10% environ à 90% environ en poids du copolymère bloc,
- le poids moléculaire moyen du copolymère bloc étant d'au moins 3.000.

7. Composition selon l'une quelconque des revendications précédentes, caractérisé en ce que la cire est choisie parmi celles qui possèdent un point de fusion compris entre 40 et 110°C

8. Composition selon l'une quelconque des revendications précédentes, caractérisé en ce que la cire est choisie parmi celles qui sont solides et rigides à température inférieure à 50 °C.

9. Composition selon l'une quelconque des revendications précédentes, caractérisée en ce que la cire est choisie parmi celles ayant une pénétration à l'aiguille, à 25 °C, comprise entre 3 et 40, telle que mesurée selon la norme américaine ASTM D 5 ou selon la norme française NFT 004.

10. Composition selon l'une quelconque des revendications précédentes, caractérisé en ce qu'elle comprend de 6 à 40% de cire, en poids par rapport au poids total de la composition, et préférentiellement de 10 à 25%.

11. Composition selon l'une quelconque des revendications précédentes, caractérisé en ce qu'elle comprend de 0,1 à 25% de polymère filmogène en poids par rapport au poids total de la composition, préférentiellement de 1 à 10%.

12. Composition selon l'une quelconque des revendications précédentes, caractérisé en ce qu'elle comprend de 0,01 à 5% d'agent siliconé en poids par rapport au poids total de la composition, et préférentiellement de 0,4 à 1,5%.

13. Composition selon l'une quelconque des revendications précédentes, caractérisé en ce qu'elle comprend de 10 à 30% d'agent siliconé en poids par rapport au poids total de polymère filmogène.

14. Composition selon l'une quelconque des revendications précédentes, caractérisé en ce qu'elle est sous forme d'une émulsion huile-dans-eau.

15. Composition selon la revendication précédente, caractérisé en ce qu'elle comprend de 2 à 30 % en poids par rapport au poids total de la composition d'au moins un agent tensioactif émulsionnant.

16. Composition selon la revendication précédente, caractérisé en ce que les tensioactifs sont choisis parmi :
les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, les stéarates d'amines.

17. Composition selon l'une quelconque des revendications 14 à 16, caractérisée en ce qu'elle comprend de 30 à 80% d'eau en poids par rapport au poids total de la composition.

18. Composition selon l'une quelconque des revendications 1 à 13, caractérisée en ce qu'elle est sous la forme d'une dispersion eau-dans-cire.

19. Composition selon la revendication précédente, caractérisée en ce qu'elle contient en outre au moins un solvant ou une huile organique volatile.

20. Composition selon l'une quelconque des revendications 18 et 19, caractérisée en ce qu'elle comprend en outre au moins un agent épaississant.

21. Composition selon l'une quelconque des revendications 18 à 20, caractérisée en ce que la phase aqueuse représente de 0,1 à 25% en poids par rapport au poids total de la composition.

22. Composition selon l'une quelconque des revendications précédentes, caractérisée en ce qu'elle constitue un mascara ou un liner ou une base pour la préparation de mascaras ou de liners.

23. Composition selon l'une quelconque des revendications précédentes, caractérisée en ce que la silicone oxyalkylénée ne contient pas de groupement alkyle ou alcoxy ayant de 8 à 22 atomes de carbone et lié directement à un atome de silicium.

24. Utilisation d'une composition selon l'une quelconque des revendications précédentes, pour l'obtention un maquillage présentant une meilleure tenue dans le temps et/ou un empesage amélioré et/ou une meilleure résistance à l'effritement et/ou un meilleur allongement des cils et/ou une vitesse de séchage améliorée.

25. Utilisation d'un agent siliconé choisi parmi :
(i) les silicones oxyalkylénées,
(ii) les copolymères blocs linéaires polysiloxane-polyoxyalkylène,
dans une composition cosmétique, notamment pour le maquillage des yeux, comprenant :
(a) au moins une phase aqueuse comprenant un polymère filmogène,
(b) au moins une phase grasse comprenant au moins une cire,
pour conférer à cette composition une meilleure tenue dans le temps et/ou un empesage amélioré et/ou une meilleure résistance à l'effritement et/ou un meilleur allongement des cils et/ou une vitesse de séchage améliorée.

26. Procédé de maquillage des cils consistant à appliquer sur les cils une composition selon l'une quelconque des revendications 1 à 23.

## Claims

1. Cosmetic composition, characterized in that it comprises:
(a) at least one aqueous phase comprising a film-forming polymer,
(b) at least one fatty phase comprising at least one wax,
(c) a silicone agent chosen from:
(i) oxyalkylenated silicones,
(ii) linear polysiloxane-polyoxyalkylene block copolymers.

2. Composition according to the preceding claim, characterized in that it is in the form of an emulsion of the fatty phase in the aqueous phase or of a dispersion of the aqueous phase in the fatty phase.

3. Composition according to either one of the preceding claims, characterized in that it additionally comprises from 0.25 to 25%, preferably from 1 to 20%, by weight with respect to the total weight of the composition of at least one pigment.

4. Composition according to any one of the preceding claims, characterized in that the oxyalkylenated silicone is chosen from the compounds of general formula (I): 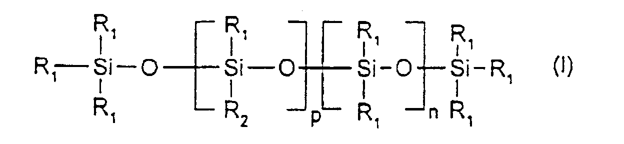 in which:
- R₁, which are identical or different, represent a hydrogen atom, a linear or branched C₁-C₃₀ alkyl radical or a phenyl radical,
- R₂, which are identical or different, represent -(CₓH₂ₓ)-(OC₂H₄)ₐ-(OC₃H₆)_{b}-OR₃,
- R₃, which are identical or different, are chosen from a hydrogen atom, a linear or branched alkyl radical having from 1 to 12 carbon atoms or a linear or branched acyl radical having from 2 to 12 carbon atoms,
- n varies from 0 to 1000,
- p varies from 1 to 30,
- a varies from 0 to 50,
- b varies from 0 to 50,
- a + b is greater than or equal to 1,
- x varies from 1 to 5,
the number-average molecular weight being greater than or equal to 15,000 and preferably between 25,000 and 75,000.

5. Composition according to the preceding claim, characterized in that it comprises an oxyalkylenated silicone of general formula (I) which corresponds to at least one and preferably all of the following conditions:
- R₁ denotes a methyl radical,
- R₃ represents a hydrogen atom, a methyl radical or an acetyl radical and preferably hydrogen,
- p varies from 8 to 20,
- a is between 5 and 40 and preferably between 15 and 30,
- b is between 5 and 40 and preferably between 15 and 30,
- x is equal to 2 or 3,
- n varies from 20 to 600, preferably from 50 to 500 and more particularly still from 100 to 300.

6. Composition according to any one of the preceding claims, characterized in that the linear polysiloxane-polyoxyalkylene block copolymer corresponds to the following formula (II):
([Y(R₂SiO)_{a'}R'₂SiYO][(C_{n'}H_{2n'}O)_{b'}])_{c'} (II)
in which:
- R and R', which are identical or different, represent a monovalent hydrocarbon-comprising radical not comprising aliphatic unsaturation,
- n' is an integer between 2 and 4,
- a' is an integer greater than or equal to 5,
- b' is an integer greater than or equal to 4,
- c' is an integer greater than or equal to 4,
- Y represents a divalent organic group which is bonded to the adjacent silicon atom via a carbon-silicon bond and to a polyoxyalkylene block via an oxygen atom,
- the average molecular weight of each siloxane block is between approximately 400 and approximately 10,000, that of each polyoxyalkylene block being between approximately 300 and approximately 10,000,
- the siloxane blocks represent from approximately 10% to approximately 90% by weight of the block copolymer,
- the average molecular weight of the block copolymer being at least 3000.

7. Composition according to any one of the preceding claims, characterized in that the wax is chosen from those which have a melting point of between 40 and 110°C.

8. Composition according to any one of the preceding claims, characterized in that the wax is chosen from those which are solid and rigid at a temperature of less than 50°C.

9. Composition according to any one of the preceding claims, characterized in that the wax is chosen from those having a needle penetration at 25°C of between 3 and 40, as measured according to United States Standard ASTM D 5 or according to French Standard NFT 004.

10. Composition according to any one of the preceding claims, characterized in that it comprises from 6 to 40% of wax by weight with respect to the total weight of the composition and preferably from 10 to 25%.

11. Composition according to any one of the preceding claims, characterized in that it comprises from 0.1 to 25% of film-forming polymer by weight with respect to the total weight of the composition and preferably from 1 to 10%.

12. Composition according to any one of the preceding claims, characterized in that it comprises from 0.01 to 5% of silicone agent by weight with respect to the total weight of the composition and preferably from 0.4 to 1.5%.

13. Composition according to any one of the preceding claims, characterized in that it comprises from 10 to 30% of silicone agent by weight with respect to the total weight of the film-forming polymer.

14. Composition according to any one of the preceding claims, characterized in that it is in the form of an oil-in-water emulsion.

15. Composition according to the preceding claim, characterized in that it comprises from 2 to 30% by weight with respect to the total weight of the composition of at least one emulsifying surface-active agent.

16. Composition according to the preceding claim, characterized in that the surfactants are chosen from: fatty acids, fatty alcohols, polyethoxylated or polyglycerolated fatty alcohols, such as polyethoxylated stearyl or cetylstearyl alcohols, fatty acid eaters of sucrose, alkyl glucose eaters, in particular polyoxyethylenated fatty eaters of C₁-C₆ alkyl glucose, or amine stearates.

17. Composition according to any one of Claims 14 to 16, characterized in that it comprises from 30 to 80% of water by weight with respect to the total weight of the composition.

18. Composition according to any one of Claims 1 to 13, characterized in that it is in the form of a water-in-wax dispersion.

19. Composition according to the preceding claim, characterized in that it additionally comprises at least one volatile organic solvent or volatile organic oil.

20. Composition according to either one of Claims 18 and 19, characterized in that it additionally comprises at least one thickening agent.

21. Composition according to any one of Claims 18 to 20, characterized in that the aqueous phase represents from 0.1 to 25% by weight with respect to the total weight of the composition.

22. Composition according to any one of the preceding claims, characterized in that it constitutes a mascara or an eyeliner or a base for the preparation of mascaras or of eyeliners.

23. Composition according to any one of the preceding claims, characterized in that the oxyalkylenated silicone does not comprise an alkyl or alkoxy group having from 8 to 22 carbon atoms bonded directly to a silicon atom.

24. Use of a composition according to any one of the preceding claims for producing a make-up exhibiting better hold over time and/or improved stiffening and/or better resistance to disintegration and/or better elongation of the eyelashes and/or an improved rate of drying.

25. Use of a silicone agent chosen from:
(i) oxyalkylenated silicones,
(ii) linear polysiloxane-polyoxyalkylene block copolymers,
in a cosmetic composition, in particular for making up the eyes, comprising:
(a) at least one aqueous phase comprising a film-forming polymer,
(b) at least one fatty phase comprising at least one wax,
for conferring, on this composition, better hold over time and/or improved stiffening and/or better resistance to disintegration and/or better elongation of the eyelashes and/or an improved rate of drying. 26. Process for making up the eyelashes which consists in applying, to the eyelashes, a composition according to any one of Claims 1 to 23.

## Patentansprüche

1. Kosmetische Zusammensetzungen, dadurch gekennzeichnet, daß sie enthalten:
a) mindestens eine wässerige Phase, die ein filmbildendes Polymer enthält,
b) mindestens eine Fettphase, die mindestens ein Wachs enthält, und
c) ein siliconhaltiges Mittel, das ausgewählt ist unter:
i) alkoxylierten Siliconen und
ii) geradkettigen Polysiloxan-Polyoxyalkylen-Blockcopolymeren.

2. Zusammensetzungen nach dem vorhergehenden Anspruch, dadurch gekennzeichnet, daß sie in Form einer Emulsion der Fettphase in der wässerigen Phase oder einer Dispersion der wässerigen Phase in der Fettphase vorliegen.

3. Zusammensetzungen nach einem der vorhergehenden Ansprüche, dadurch gekennzeichnet, daß sie ferner 0,25 bis 25 Gew.-% und vorzugsweise 1 bis 20 Gew.=% mindestens eines Pigments, bezogen auf das Gesamtgewicht der Zusammensetzung, enthalten.

4. Zusammensetzungen nach einem der vorhergehenden Ansprüche, dadurch gekennzeichnet, daß das alkoxylierte Silicon ausgewählt ist unter den Verbindungen der allgemeinen Formel (I): 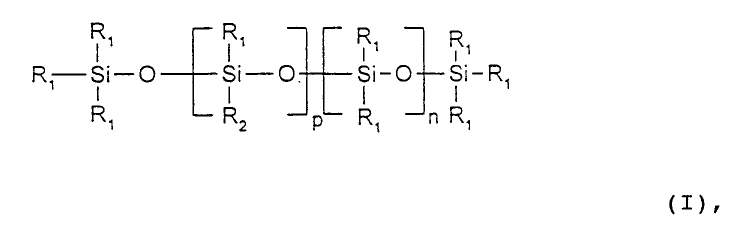 worin:
- die Gruppen R₁, die identisch oder voneinander verschieden sind, ein Wasserstoffatom, eine geradkettige oder verzweigte C₁₋₃₀-Alkylgruppe oder eine Phenylgruppe bedeuten,
- die Gruppen R₂, die identisch oder voneinander verschieden sind, -(CₓH₂ₓ)-(OC₂H₄)ₐ-(OC₃H₆)_{b}-OR₃ bedeuten,
- die Gruppen R₃, die identisch oder voneinander verschieden sind, unter einem Wasserstoffatom, einer geradkettigen oder verzweigten Alkylgruppe mit 1 bis 12 Kohlenstoffatomen und einer geradkettigen oder verzweigten Acylgruppe mit 2 bis 12 Kohlenstoffatomen ausgewählt sind,
- n im Bereich von 0 bis 1 000 liegt,
- p im Bereich von 1 bis 30 liegt,
- a im Bereich von 0 bis 50 liegt,
- b im Bereich von 0 bis 50 liegt,
- a + b größer oder gleich 1 ist und
- X im Bereich von 1 bis 5 liegt,
wobei das Zahlenmittel des Molekulargewichts mindestens 15 000 beträgt und vorzugsweise im Bereich von 25 000 bis 75 000 liegt.

5. Zusammensetzungen nach dem vorhergehenden Anspruch, dadurch gekennzeichnet, daß sie ein alkoxyliertes Silicon der allgemeinen Formel (I) enthalten, das mindestens eine und vorzugsweise sämtliche der folgenden Bedingungen erfüllt:
- R₁ bedeutet Methyl.
- R₃ bedeutet ein Wasserstoffatom, eine Methylgruppe oder eine Acetylgruppe und vorzugsweise Wasserstoff.
- p liegt im Bereich von 8 bis 20.
- a liegt im Bereich von 5 bis 40 und vorzugsweise von 15 bis 30.
- b liegt im Bereich von 5 bis 40 und vorzugsweise von 15 bis 30.
- X ist gleich 2 oder 3.
- n liegt im Bereich von 20 bis 600, vorzugsweise von 50 bis 500 und noch bevorzugter von 100 bis 300.

6. Zusammensetzungen nach einem der vorhergehenden Ansprüche, dadurch gekennzeichnet, daß das geradkettige Polysiloxan-Polyoxyalkylen-Blockcopolymer der folgenden Formel (II) entspricht:
([Y(R₂SiO)_{a'}R'₂SiYO][(C_{n'}H_{2n'}O)_{b'}])_{c'} (II)
, worin:
- R und R', die identisch oder voneinander verschieden sind, eine einwertige kohlenwasserstoffhaltige Gruppe bedeuten, die keine aliphatisch ungesättigte Doppelbindung enthält,
- n' eine ganze Zahl im Bereich von 2 bis 4 bedeutet,
- a' eine ganze Zahl bedeutet, die größer oder gleich 5 ist,
- b' eine ganze Zahl bedeutet, die größer oder gleich 4 ist,
- c' eine ganze Zahl bedeutet, die größer oder gleich 4 ist,
- Y eine zweiwertige organische Gruppe bedeutet, die über eine Kohlenstoff-Silicium-Bindung an das angrenzende Siliciumatom und über ein Sauerstoffatom an einen Polyoxyalkylenblock gebunden ist,
- das mittlere Molekulargewicht der einzelnen Siloxanblöcke im Bereich von ungefähr 400 bis ungefähr 10 000 liegt, wobei das mittlere Molekulargewicht der einzelnen Polyoxyalkylenblöcke im Bereich von ungefähr 300 bis ungefähr 10 000 liegt, und
- die Siloxanblöcke etwa 10 Gew.-% bis etwa 90 Gew.-% des Blockcopolymers ausmachen,
- wobei das mittlere Molekulargewicht des Blockcopolymers mindestens 3 000 beträgt.

7. Zusammensetzungen nach einem der vorhergehenden Ansprüche, dadurch gekennzeichnet, daß das Wachs unter den Wachsen ausgewählt ist, die einen Schmelzpunkt im Bereich von 40 bis 110 °C aufweisen.

8. Zusammensetzungen nach einem der vorhergehenden Ansprüche, dadurch gekennzeichnet, daß das Wachs unter den Wachsen ausgewählt ist, die bei einer Temperatur unter 50 °C fest und hart sind.

9. Zusammensetzungen nach einem der vorhergehenden Ansprüche, dadurch gekennzeichnet, daß das Wachs unter den Wachsen ausgewählt ist, die eine nach der amerikanischen Norm ASTM D 5 oder nach der französischen Norm NFT 004 bestimmte Nadeleindringtiefe bei 25 °C im Bereich von 3 bis 40 aufweisen.

10. Zusammensetzungen nach einem der vorhergehenden Ansprüche, dadurch gekennzeichnet, daß sie 6 bis 40 Gew.-% und vorzugsweise 10 bis 25 % Wachs, bezogen auf das Gesamtgewicht der Zusammensetzung, enthalten.

11. Zusammensetzungen nach einem der vorhergehenden Ansprüche, dadurch gekennzeichnet, daß sie 0,1 bis 25 Gew.-% und vorzugsweise 1 bis 10 Gew.-% filmbildendes Polymer, bezogen auf das Gesamtgewicht der Zusammensetzung, enthalten.

12. Zusammensetzungen nach einem der vorhergehenden Ansprüche, dadurch gekennzeichnet, daß sie 0,01 bis 5 Gew.-% und vorzugsweise 0,4 bis 1,5 Gew.-% siliconhaltiges Mittel, bezogen auf das Gesamtgewicht der Zusammensetzung, enthalten.

13. Zusammensetzungen nach einem der vorhergehenden Ansprüche, dadurch gekennzeichnet, daß sie 10 bis 30 Gew.-% siliconhaltiges Mittel, bezogen auf das Gesamtgewicht des filmbildenden Polymers, enthalten.

14. Zusammensetzungen nach einem der vorhergehenden Ansprüche, dadurch gekennzeichnet, daß sie in Form einer Ölin-Wasser-Emulsion vorliegen.

15. Zusammensetzungen nach dem vorhergehenden Anspruch, dadurch gekennzeichnet, daß sie 2 bis 30 Gew.-% mindestens eines emulgierenden grenzflächenaktiven Stoffs, bezogen auf das Gesamtgewicht der Zusammensetzung, enthalten.

16. Zusammensetzungen nach dem vorhergehenden Anspruch, dadurch gekennzeichnet, daß die grenzflächenaktiven Stoffe ausgewählt sind unter: Fettsäuren, Fettalkoholen, polyethoxylierten oder mehrfach veretherten Fettalkoholen, wie polyethoxyliertem Stearylalkohol oder polyethoxyliertem Cetylstearylalkohol, Fettsäure-Saccharose-Estern, Alkylglucoseestern, insbesondere polyethoxylierten C₁₋₆-Alkylglucosefettestern, und Aminstearaten.

17. Zusammensetzungen nach einem der Ansprüche 14 bis 16, dadurch gekennzeichnet, daß sie 30 bis 80 Gew.-% Wasser, bezogen auf das Gesamtgewicht der Zusammensetzung, enthalten.

18. Zusammensetzungen nach einem der Ansprüche 1 bis 13, dadurch gekennzeichnet, daß sie in Form einer Wasser-in-Wachs-Dispersion vorliegen.

19. Zusammensetzungen nach dem vorhergehenden Anspruch, dadurch gekennzeichnet, daß sie ferner mindestens ein flüchtiges organisches Lösemittel oder Öl enthalten.

20. Zusammensetzungen nach einem der Ansprüche 18 und 19, dadurch gekennzeichnet, daß sie ferner mindestens ein Verdickungsmittel enthalten.

21. Zusammensetzungen nach einem der Ansprüche 18 bis 20, dadurch gekennzeichnet, daß die wässerige Phase 0,1 bis 25 Gew.-%, bezogen auf das Gesamtgewicht der Zusammensetzung, ausmacht.

22. Zusammensetzungen nach einem der vorhergehenden Ansprüche, dadurch gekennzeichnet, daß sie ein Mascara oder ein Eyeliner oder eine Grundmasse zur Herstellung von Mascaras oder Eyelinern sind.

23. Zusammensetzungen nach einem der vorhergehenden Ansprüche, dadurch gekennzeichnet, daß das alkoxylierte Silicon keine Alkylgruppe oder Alkoxygruppe mit 8 bis 22 Kohlenstoffatomen enthält, die direkt an ein Siliciumatom gebunden ist.

24. Verwendung einer Zusammensetzung nach einem der vorhergehenden Ansprüche zur Herstellung einer Schminke, die länger haltbar ist und/oder die Wimpern besser stärkt und/oder beständiger gegenüber Abbröckeln ist und/oder die Wimpern stärker verlängert und/oder schneller trocknet.

25. Verwendung eines siliconhaltigen Mittels, das ausgewählt ist unter:
i) alkoxylierten Siliconen und
ii) geradkettigen Polysiloxan-Polyoxyalkylen-Blockcopolymeren,
in kosmetischen Zusammensetzungen und insbesondere in Zusammensetzungen zum Schminken der Augen, die enthalten:
a) mindestens eine wässerige Phase, die ein filmbildendes Polymer enthält, und
b) mindestens eine Fettphase, die mindestens ein Wachs enthält,
damit die Zusammensetzungen länger haltbar sind und/oder die Wimpern besser stärken und/oder beständiger gegenüber Abbröckeln sind und/oder die Wimpern stärker verlängern und/oder schneller trocknen.

26. Verfahren zum Schminken der Wimpern, das darin besteht, eine Zusammensetzung nach einem der Ansprüche 1 bis 23 auf die Wimpern aufzutragen.
